# FASCICULE DE BREVET EUROPEEN

(11) **EP 3 048 890 B1**
(45) Date de publication et mention de la délivrance du brevet: **18.04.2018**
(21) Numéro de dépôt: 14790658.0
(22) Date de dépôt: 24.09.2014
(51) Int. Cl.: A01N 63/00, A01N 63/02, C12N 1/20, C12R 1/465, C05F 11/08

(54) **BACTÉRIE ISOLÉE DU GENRE STREPTOMYCES**
ISOLIERTES BAKTERIUM DER GATTUNG STREPTOMYCES
ISOLATED BACTERIUM OF THE GENUS STREPTOMYCES

(30) Priorité: 24.09.2013 FR 1359186
(43) Date de publication de la demande: 03.08.2016
(73) Titulaire: Agronutrition, 31390 Carbonne (FR)
(72) Inventeur: ERRAKHI, Rafik, Benguerir (MA); ATTIA, Faouzi, F-31450 Ayguesvives (FR); CABANES, Cédric, F-31320 Pechabou (FR)
(74) Mandataire: Cabinet BARRE LAFORGUE & associés
(86) Numéro de dépôt international: PCT/FR2014/052387
(87) Numéro de publication internationale: WO 2015/044584

(56) Documents cités:
- WO-A2-2009/156687
- WO-A2-2009/156688
- WO-A2-2012/016140
- DE-A1- 10 324 193
- KR-A- 20100 055 364
- DATABASE EMBL [Online] 31 janvier 2013 (2013-01-31), "Streptomyces sp. AgN23 16S ribosomal RNA gene, partial sequence.", XP002722625, extrait de EBI accession no. EMBL:JN104730 Database accession no. JN104730
- HYE YOON KIM ET AL: "Identification of antifungal niphimycin from Streptomyces sp. KP6107 by screening based on adenylate kinase assay", JOURNAL OF BASIC MICROBIOLOGY, vol. 53, no. 7, 23 juillet 2013 (2013-07-23), pages 581-589, XP055111207, ISSN: 0233-111X, DOI: 10.1002/jobm.201200045 -& DATABASE EMBL [Online] 15 mai 2011 (2011-05-15), "Streptomyces hygroscopicus subsp. enhygrus strain KP6107 16S ribosomal RNA gene, partial sequence.", XP002722624, extrait de EBI accession no. EMBL:HQ441251 Database accession no. HQ441251
- SOUAD LOQMAN ET AL: "Antagonistic actinomycetes from Moroccan soil to control the grapevine gray mold", WORLD JOURNAL OF MICROBIOLOGY AND BIOTECHNOLOGY, KLUWER ACADEMIC PUBLISHERS, DO, vol. 25, no. 1, 4 octobre 2008 (2008-10-04), pages 81-91, XP019650434, ISSN: 1573-0972
- S SRIVIDYA ET AL: "Streptomyces sp. 9p as effective biocontrol against chilli soilborne fungal phytopathogens", EUROPEAN JOURNAL OF EXPERIMENTAL BIOLOGY, vol. 2, no. 1, 2012, pages 163-173, XP055111252,
- S. LOQMAN ET AL: "Streptomyces thinghirensis sp. nov., isolated from rhizosphere soil of Vitis vinifera", INTERNATIONAL JOURNAL OF SYSTEMATIC AND EVOLUTIONARY MICROBIOLOGY, vol. 59, no. 12, 30 juillet 2009 (2009-07-30), pages 3063-3067, XP055164561, ISSN: 1466-5026, DOI: 10.1099/ijs.0.008946-0
- Q. TU ET AL: "Strain/Species-Specific Probe Design for Microbial Identification Microarrays", APPLIED AND ENVIRONMENTAL MICROBIOLOGY, vol. 79, no. 16, 7 juin 2013 (2013-06-07), pages 5085-5088, XP055111359, ISSN: 0099-2240, DOI: 10.1128/AEM.01124-13
- KOJI FUKUDA ET AL: "Production improvement of antifungal, antitrypanosomal nucleoside sinefungin by rpoB mutation and optimization of resting cell system of Streptomyces incarnatus NRRL 8089", JOURNAL OF BIOSCIENCE AND BIOENGINEERING, vol. 109, no. 5, mai 2010 (2010-05), pages 459-465, XP055164478, ISSN: 1389-1723, DOI: 10.1016/j.jbiosc.2009.10.017 -& DATABASE EMBL [Online] 10 février 2010 (2010-02-10), "Streptomyces incarnatus rpoB gene for RNA polymerase beta-subunit, complete cds.", XP002734967, extrait de EBI accession no. EMBL:AB516306 Database accession no. AB516306
- MIKA T. TARKKA ET AL: "Plant behavior upon contact with streptomycetes", PLANT SIGNALING & BEHAVIOR, vol. 3, no. 11, novembre 2008 (2008-11), pages 917-919, XP055164498, DOI: 10.4161/psb.5996

### SEQUENCE LISTING

<110> AGRONUTRITION
<120> NOUVELLE SOUCHE DE STREPTOMYCES
<150> FR1359186
   <151> 2013-09-24
<160> 8
<170> BiSSAP 1.0
<210> 1
   <211> 1336
   <212> DNA
   <213> Streptomyces
<220>
   <221> source
   <222> 1..1336
   <223> /mol_type="DNA" /organism="Streptomyces"
<400> 1
   tagtggcgaa cgggtgagta acacgtgggc aatctgccct gcactctggg acaagccctg      60
   gaaacggggt ctaataccgg atatgacacg ctcccgcatg ggatgcgtgt ggaaagctcc     120
   ggcggtgcag gatgagcccg cggcctatca gcttgttggt ggggtgatgg cctaccaagg     180
   cgacgacggg tagccggcct gagagggcga ccggccacac tgggactgag acacggccca     240
   gactcctacg ggaggcagca gtggggaata ttgcacaatg ggcgaaagcc tgatgcagcg     300
   acgccgcgtg agggatgacg gccttcgggt tgtaaacctc tttcagcagg gaagaagcga     360
   gagtgacggt acctgcagaa gaagcgccgg ctaactacgt gccagcagcc gcggtaatac     420
   gtagggcgca agcgttgtcc ggaattattg ggcgtaaaga gctcgtaggc ggcttgtcgc     480
   gtcggatgtg aaagcccggg gcttaacccc gggtctgcat tcgatacggg caggctagag     540
   ttcggtaggg gagatcggaa ttcctggtgt agcggtgaaa tgcgcagata tcaggaggaa     600
   caccggtggc gaaggcggat ctctgggccg atactgacgc tgaggagcga aagcgtgggg     660
   agcgaacagg attagatacc ctggtagtcc acgccgtaaa cgttgggaac taggtgtggg     720
   cgacattcca cgtcgtccgc gccgcagcta acgcattaag ttccccgcct ggggagtacg     780
   gccgcaaggc taaaactcaa aggaattgac ggggggcccg cacaagcggc ggagcatgtg      840
   gcttaattcg acgcaacgcg aagaacctta ccaaggcttg acatacaccc ggaaacctct     900
   ggagacaggg gccccccttg tggtcggtgt acaggtggtg catggcttgt cgtcagctcg      960
   tgtcgtgaga tgttgggtta agtccccgca acgagcgcaa cccttgttct gtgttgccag    1020
   catgcctttc gggggntgat ggggacttnc acaggagact gccggggtca actcggagga    1080
   aggtggggac gacgtcaagt catcatgccc cttatgtctt gggctgcaca cgtgctacaa    1140
   tggccggtac aatgagctgc gaagccgtga ggtggagcga atctcaaaaa gccggtctca 1200
   gttcggattg gggtctgcaa ctcgacccca tgaagtcgga gtcgctagta atcgcagatc 1260
   agcattgctg cggtgaatac gttcccgggc cttgtacaca ccgcccgtca cgtcacgaaa 1320
   gtcggtaaca cctgaa 1336
<210> 2
   <211> 20
   <212> DNA
   <213> Streptomyces
<220>
   <221> source
   <222> 1..20
   <223> /mol_type="DNA" /organism="Streptomyces"
<400> 2
   agagtttgat cctggctcag 20
<210> 3
   <211> 19
   <212> DNA
   <213> Streptomyces
<220>
   <221> source
   <222> 1..19
   <223> /mol_type="DNA" /note="Amorce 1429r" /organism="Streptomyces"
<400> 3
   ggttaccttg ttacgactt 19
<210> 4
   <211> 3483
   <212> DNA
   <213> Streptomyces
<220>
   <221> source
   <222> 1..3483
   <223> /mol_type="DNA" /organism="Streptomyces" /plasmid="rpoB"
<400> 4
   ttggccgcct cgcgcaacgc ctcgactgcc aatacgaaca atggcgccag caccgccccg 60
   ctgcgcatct cctttgcgaa gatcagggag cctctcgagg ttccgaacct cctcgcgctg 120
   cagaccgaga gcttcgattg gctgctcggc aatgccgcct ggaaggctcg cgtcgaggct 180
   gcgctggaca gcggtcagga cgtccccacc aagtccggtc tggaagagat cttcgaggag 240
   atctccccga tcgaggactt ctccgggtcg atgtccctga ctttccgtga tcaccgtttc 300
   gagccgccga agaactcgat cgacgagtgc aaggagcgtg acttcaccta cgccgctccg      360
   ctcttcgtca cggccgagtt caccaacaac gagaccggcg agatcaagtc ccagacggtc      420
   ttcatgggcg acttcccgct catgaccgac aagggcacct tctgcatcaa cggcaccgag      480
   cgtgtcgtcg tctcgcagct ggtccgctcg ccgggtgtct acttcgactc ctccatcgac      540
   aagacgtccg acaaggacat cttctccgtc aaggtcatcc cgtcccgggg tgcctggctg      600
   gagatggaga tcgacaagcg tgacatggtc ggtgtgcgta tcgaccgcaa gcgcaagcag      660
   tccgtcaccg ttctcctgaa ggctctcggc tggacgaccg agcagatcct ggaggagttc      720
   ggcgagtacg agtcgatgcg cgccaccctg gagaaggacc acacccaggg ccaggacgac      780
   gcgctgctcg acatctaccg caagctgcgt ccgggcgagc cccccacacg ggaggccgcg      840
   cagacgctgc tcgagaacct ctacttcaac ccgaagcgct acgacctcgc gaaggtcggc      900
   cgctacaagg tcaacaagaa gctgggttcg gccgctccgc tggacgcggg cgtcctgacg      960
   gtcgaggacg tcatcgcctc gatcaagtac ctggtgaagc tgcacgccgg tgagaccgag     1020
   accgtcgggg acaacggcca gtccgtggtc gtcgagaccg acgacatcga ccacttcggc     1080
   aaccgccgta tccgtaacgt cggcgagctg atccagaacc aggtccgcac gggtctggcc     1140
   cgtatggagc gcgtcgtgcg tgagcgcatg acgactcagg acgtcgaggc gatcacgccg     1200
   cagaccctga tcaacatccg gccggtcgtc gcctccatca aggagttctt cggcaccagc     1260
   cagctgtcgc agttcatgga ccagacgaac ccgctgtcgg gtctgaccca caagcgccgt     1320
   ctgaacgcgc tcggccccgg tggtctctcc cgtgagcggg cgggcttcga ggtccgtgac     1380
   gtgcacccgt cgcactacgg ccgcatgtgc ccgatcgaga cgcccgaagg cccgaacatc     1440
   ggtctgatcg gctcgctcgc ctcgtacggc cgggtcaacg cgttcggttt catcgagacc     1500
   ccgtaccgca aggtcgtcga cggtgtcgtc accgacgacg tcgactacct gacggccgat     1560
   gaagaggacc gcttcgtcat cgcgcaggcc aacgccccgc tcgcggacga cctgcgcttc     1620
   gccgagaacc gcgtcctggt ccgccgccgt ggcggcgagg tcgactacat ccccggcgac     1680
   gacgtcgact acatggacgt ctcaccgcgc cagatggtgt cggtcgcgac cgcgatgatc     1740
   cccttcctcg agcacgacga cgccaaccgc gcgctcatgg gctcgaacat gatgcgccag     1800
   gccgtgccgc tgatcaaggc ggagtccccg ctggtcggca ccggcatgga gtaccgctgt     1860
   gcggtcgacg ccggcgacgt catcaaggcc gagaaggacg gtgtcgtcca ggaggtctcc     1920
   gccgactacg tgacggtggc caacgacgac ggcacctaca ccacctaccg ggtggccaag     1980
   ttctcccgct ccaaccaggg cacctccttc aaccagaagg tcgtcgtgga cgagggtgcg     2040
   cgggtgatcg ccggccaggt gctggccgac ggcccgtcca ccgaggacgg cgagatggcg     2100
   ctcggcaaga acctcctggt ggcgttcatg ccgtgggagg gccacaacta cgaggacgcg     2160
   atcatcctca gccagcgtct ggtgcaggac gacgtcctct cctcgatcca catcgaggag     2220
   cacgaggtcg atgcccgtga caccaagctc ggccccgagg agatcacccg ggacatcccg    2280
   aacgtctccg aggaggtcct cgccgacctc gacgagcgcg gcatcatccg gatcggtgcc    2340
   gaggtcgtcg ccggcgacat cctggtcggc aaggtcaccc cgaagggcga gaccgagctg    2400
   accccggagg agcggctgct gcgcgcgatc ttcggtgaga aggcccgtga ggtccgtgac    2460
   acctcgctga aggtgccgca cggtgagatc ggcaaggtca tcggcgtccg cgtcttcgac    2520
   cgcgaagagg gcgacgaact gccgccgggc gtgaaccagc tggtccgcgt ctacgtggcg    2580
   cagaagcgca agatcaccga tggtgacaag ctcgccggcc gtcacggcaa caagggcgtc    2640
   atctccaaga tcctgccggt cgaggacatg ccgttcctgg aggacggcac cccggtcgac    2700
   atcatcctca acccgctggg tgtcccgtcc cgaatgaacc cgggacaggt cctggagatc    2760
   cacctgggct ggctggcctc ccgcggctgg aaggtcgagg gctccgagga ctggatgcag    2820
   cggctccagg ccatcggcgc cgacgaggtc gagcccggca ccaacgtcgc gaccccggtc    2880
   ttcgacggcg cccgcgagga cgagatcgcc ggtctcttcg actcgacgat cccgaaccgc    2940
   gacggcgacc gcctggtcca gtcgtccggc aaggcccggc tcttcgacgg ccgctccggc    3000
   gagccgttcc cggagccgat ctcggtcggc tacatgtaca tcctcaagct gcaccacctg    3060
   gtggacgaca agctgcacgc ccggtccacc ggtccgtact cgatgatcac ccagcagccg    3120
   ctgggtggta aggctcagtt cggtggccag cgcttcggtg agatggaggt gtgggcgctg    3180
   gaggcttatg gcgccgcgta cgccctccag gagctgctga ccatcaagtc cgacgacgtg    3240
   accggccgcg tgaaggtcta cgaggccatc gtcaagggcg agaacattcc cgagcccggc    3300
   atccccgagt ccttcaaggt gctcatcaag gagatgcagt ccctgtgcct caacgtggag    3360
   gtgctgtcgt ccgacggcat gtccatcgag atgcgcgaca ccgacgagga cgtcttccgc    3420
   gctgcggagg agctcggtat cgacctgtcc cggcgcgagc cgagcagcgt cgaagaggtc    3480
   tga                                                                  3483
<210> 5
   <211> 2031
   <212> DNA
   <213> Streptomyces
<220>
   <221> source
   <222> 1..2031
   <223> /mol_type="DNA" /note="gyr B" /organism="Streptomyces"
<400> 5
   gtgctgtgcc agaaagggcg cttcgtggcc gattccggca accccatcga aaacatcccg      60
   tccacgcccg acgacgaggc cctggctccg ccgtcgtacg acgccagtgc gattaccgtc     120
   ctggaagggc tggaggcggt ccgcaagcga cccggtatgt acatcggttc caccggtgag     180
   cgcggcctgc accatctcgt ccaagaggtc gtcgacaact ccgtcgacga ggccatggcc     240
   ggtcacgcgg acagcatcga ggtcacgatc ctcgccgacg gcggcgtccg cgtcgtggac     300
   aacggccgcg ggatcccggt gggcatcgtc ccctcggagg ggaagccggc tgtggaggtc     360
   gtgctgaccg tgctgcacgc gggcggcaag ttcggcggcg gcggctacgc cgtctccggc     420
   ggtctgcacg gcgtcggcgt ctccgtcgtc aacgccctgt cctcgaaggt gtcggtcgag     480
   gtcaagacgg acggctaccg ctggacccag gactacaaga cgggagcgcc gaccgcgccc     540
   ctggcccgga acgaggccac ggaggagacc ggcaccacgg tcaccttctg ggcggacccg     600
   gacgtcttcg agaccaccga gtactccttc gagacgctgg cccggcgctt ccaggagatg     660
   gcgttcctca acaagggcct gtcgatctcg ctcaaggacg agcgcgaggc ccatgtggac     720
   gaggagggca agccgctctc cgtgaagtac cactacgagg gcggcatcgt cgacttcgtg     780
   acctacctca actcccgcaa gggcgagctg gtccacccca cggtgatcgg gttcgaggcc     840
   gaggacaagg agcggatgct ctccctcgag atcgcgatgc agtggaacac ccagtacacc     900
   gagggtgtct acagcttcgc gaacaccatc cacacccatg agggcggcac ccacgaggag     960
   ggcttccgcg ccgcgctgac gtacctgatc aacaagtacg cgcgcgacaa gaagctgctc    1020
   cgggagcgtg acgacaacct caccggtgag gacatccgcg agggcctgac cgccatcatc    1080
   tcggtcaagc tgggcgagcc gcagttcgag ggccagacca agaccaagct gggcaacacg    1140
   gaggccaaga ccttcgtcca gaagatcgtc aacgagcatc tcgccgactg gctggaccgt    1200
   aaccctaatg aggcggcgga catcgtccgc aaggggatcc aggcggcgac ggcccgggtc    1260
   gcggcccgta aggcgcggga tctgacccgc cgtaaggggc tgctggagac cgcgtcgctg    1320
   ccgggcaagc tgagcgactg ccagtccaat gacccgtcga agtgcgagat cttcatcgtc    1380
   gagggtgact ccgccggcgg ctcggccaag tccggccgta acccgcagta tcaggcgatc    1440
   ctcccgatcc gcggcaagat cctcaacgtg gagaaggccc gggtcgacaa gatcctgcag    1500
   aacaacgagg tccaggcgct gatctccgcc ttcggcaccg gggtgcacga ggacttcgac    1560
   atcgccaagc tccgctatca caagatcatt ctgatggcgg acgccgatgt cgacggccag    1620
   cacatcaaca ccctgctgct gaccttcctc ttccgcttca tgcgcccgct ggtcgaggcg    1680
   gggcatgtct tcctctcccg tccgccgctc tacaagatca agtggggccg ggacgacttc    1740
   gagtacgcgt actcggaccg ggagcgggac gcgctgatcc aggtcggccg tgaacagggc    1800
   aagcgcatca gggacgactc ggtccagcgc ttcaagggtc tgggcgagat gaacgccgaa    1860
   gagctgcggg tcaccacgat ggaccccgac caccgcgtcc tgggccaggt caccctggac    1920
   gacgcggcgc aggccgacga cctgttctcg gtcctgatgg gtgaggacgt cgaggcacgg    1980
   cgctcgttca tccagcgcaa cgccaaggat gtccgcttcc tcgacatctg a             2031
<210> 6
   <211> 990
   <212> DNA
   <213> Streptomyces
<220>
   <221> source
   <222> 1..990
   <223> /mol_type="DNA" /note="Rec A" /organism="Streptomyces"
<400> 6
   ggcgatcggc cgaacgagcc ggtcgaggtc atccccaccg ggtcgaccgc tctcgacgtc      60
   gccctcggcg tcggcggtct gccgcgcggc cgggtggtcg aggtctacgg ccccgagtcc     120
   tccggtaaga cgaccctgac cctgcacgcg gtggccaatg cccagcgggc cggcggcacc     180
   gttgccttcg tggacgccga gcacgccctc gaccctgact acgcgcagaa gctgggcgtg     240
   gacaccgact ccctgatcct gtcccagccg gacaacggcg agcaggcgct cgagatcgtg     300
   gacatgctgg tccgctccgg cgccctcgac ctcatcgtca tcgactccgt cgccgccctg     360
   gtgccgcgcg cggagatcga gggcgagatg ggcgactccc acgtcggcct ccaggcccgg     420
   ctgatgagcc aggcgctccg taagatcacc agcgcgctca accagtccaa gaccaccgcg     480
   atcttcatca accagctccg cgagaagatc ggcgtgatgt tcggctcgcc ggagaccacg     540
   accggtggcc gggcgctgaa gttctacgcg tcggtgcgca tcgacatccg ccgcatcgag     600
   accctcaagg acggcaccga cgcggtcggc aaccgcaccc gcgtcaaggt cgtcaagaac     660
   aaggtcgcgc cgcccttcaa gcaggccgag ttcgacatcc tctacggcca gggcatcagc     720
   cgtgagggcg gtctgatcga catgggcgtc gagcacggct tcgtccgcaa gtccggtgcc     780
   tggtacacct acgagggcga ccagctcggc cagggcaagg agaacgcccg caacttcctg     840
   aaggacaacc ccgatctcgc caatgagatc gagaagaaga tcaaggaaaa gctcggcatc     900
   ggggtgaggc cccaggaccc ggcggccgcg gcacccacca cggacgcggc tggtgccgcg     960
   ggcgtgaccg acgccgcacc ggcgaaggcc                                      990
<210> 7
   <211> 1209
   <212> DNA
   <213> Streptomyces
<220>
   <221> source
   <222> 1..1209
   <223> /mol_type="DNA" /note="TrpB" /organism="Streptomyces"
<400> 7
   gtgcccagcg ccgagggcta tttcggcgcc ttcggcggca agttcatccc cgaggcgctc      60
   gtcgccgccg tcgacgaggt cgcggccgag tacgagaagg ccaagacgga ccccgccttc     120
   gcggccgagc tcgaggatct gctggtcaac tacaccggcc ggcccagtgc gctgaccgag     180
   gtgcggcggt tcgccgagca cgccgggggc gcccgggtct tcctcaagcg ggaggacctc     240
   aaccacaccg gctcccacaa gatcaacaat gtgctggggc aggccctgct caccaagcgc     300
   atgggcaagt cccgggtcat cgccgagacc ggcgccggtc agcacggcgt ggccacggcc     360
   accgcatgtg cgctgttcgg gctcgaatgc accatctaca tgggcgaggt cgacacccag     420
   cggcaggcgc tcaatgtggc gcggatgcgg atgctgggcg ccgaggtcat ctccgtgacc     480
   tccggcagcc gcaccctgaa ggacgccatc aacgaggcgt tccgggactg ggtcgccaat     540
   gtggaccgca cccactacct cttcggtacg gtggccggcc cccacccctt cccggcgctg     600
   gtgcgcgact tccaccgggt gatcggcgtg gaggcgcggc ggcagatcct ggagcggacc     660
   gggcggctgc cggacgcggt cgcggcctgt gtgggcggcg gatccaacgc gatcgggctg     720
   ttccacgcct tcctgccgga cgagagcgtg cgcctcgtcg gcttcgagcc cgccggacac     780
   ggtgtggaga ccggggagca cgcggccacg ctgagccagg gcgagcccgg gatcctgcac     840
   ggctcccggt cgttcgtgct ccaggacgag gacggccaga tcaccgagcc gtactcgatc     900
   tcggccggtc tcgactaccc cggcgtcggg ccggagcacg cgtatctgaa ggacatcggc     960
   cgtgccgagt accgggcggt caccgacgac gaggcgatgc gggcgctgcg gctgctctcg    1020
   gagaccgagg gcatcatccc ggcgatcgag agcgcccacg cgctggcggg cgccctggac    1080
   ctcggccgtg agctggggag cgacggcctg gtgctggtca acctctccgg gcgcggcgac    1140
   aaggacatgg acacggcggc tcggtacttc gggctctacg accagcagag cgaccaggga    1200
   gcgaagtga                                                             1209
<210> 8
   <211> 1419
   <212> DNA
   <213> Streptomyces
<220>
   <221> source
   <222> 1..1419
   <223> /mol_type="DNA" /note="AtpD" /organism="Streptomyces"
<400> 8
   gtggcctccg gccgcgtcgc gcgggtcatc ggcccggtcg tcgacgtgga gttccccgtc      60
   gacgcgatgc cggagatcta caacgcgctg caggtcgagg tcgccgaccc ctcccaggag     120
   ggggcgaaga agaccctgac cctcgaggtc gcccagcacc tcggcgaggg cctggtccgc     180
   gccatctcca tggagcccac cgacggcctg gtccgccagg ccgcggtgac cgacaccggc     240
   gacggcatca cggtgccggt cggcgatgtc accaagggcc gggtgttcaa caccctcggc     300
   aagatcctca acgagcccga ggccgagtcc gaggtcaccg agcgctggtc catccaccgc     360
   aaggccccgg ccttcgacca gctcgagtcc aagaccgaga tgttcgagac cggcctgaag     420
   gtcgtcgacc tgctgacccc gtacgtcaag ggcggcaaga tcggtctgtt cggcggcgcg     480
   ggcgtcggca agaccgtgct catccaggaa atgatcatgc gtgtggccaa gctgcacgag     540
   ggcgtttccg tgttcgccgg tgtcggcgag cgcacccgtg agggcaacga cctgatcgag     600
   gagatggccg agtccggcgt gctcccgcag accgcgctgg tcttcggcca gatggatgag     660
   cccccgggca cccgtctgcg cgtcgccctg gccggtctga ccatggcgga gtacttccgc     720
   gatgtgcaga agcaggacgt gctgttcttc atcgacaaca tcttccgctt cacccaggcc     780
   ggttccgagg tctcgaccct gctcggccgg atgccctccg cggtgggcta ccagccgaac     840
   ctggccgacg agatgggcat cctgcaggag cgcatcacct cgacccgtgg tcactcgatc     900
   acctcgatgc aggcgatcta cgtccccgcg gacgacctga ccgacccggc cccggcgacc     960
   accttcgcgc acctcgacgc gaccacggtg ctctcccggc cgatctcgga gaagggcatc    1020
   tacccggcgg tggacccgct ggactcgacg tcccggatcc tggacccgcg ctacatctcg    1080
   caggagcact acgactgcgc ctcgcgcgtg aagtcgatcc tgcagaagta caaggacctc    1140
   caggacatca tcaacatcct gggcatcgac gagctcggcg aggaggacaa gctcaccgtc    1200
   ttccgcgccc gccggatcga gcgcttcctg tcgcagaaca cccacgcggc gaagcagttc    1260
   accggcctcg acggatcgga tgtgccgctg gacgagtcca tcgccgcgtt caacgcgatc    1320
   gccgatggtg agttcgacca cttccccgag caggcgttct tcatgtgcgg tggcctggac    1380
   gacctcaagg ccaaggccaa ggagctgggc gtctcctga                           1419

## Description

L'invention concerne une nouvelle bactérie isolée du genre *Streptomyces,* une composition comprenant au moins une telle bactérie et une composition acellulaire susceptible d'être obtenue par culture d'au moins une telle bactérie.

Le développement de cultures végétales agricoles intensives est aujourd'hui nécessaire pour satisfaire les besoins alimentaires dans le monde. Ces cultures intensives nécessitent l'utilisation d'agents aptes à optimiser la nutrition des végétaux d'intérêt agricole, à favoriser leur croissance et à minimiser la croissance de végétaux et/ou d'agents pathogènes indésirables. En particulier, on utilise des agents visant à optimiser la nutrition des végétaux, des agents de protection des végétaux vis-à-vis d'organismes pathogènes, notamment vis-à-vis de bactéries phyto-pathogènes et/ou de champignons qui sont susceptibles d'affecter la croissance optimale des végétaux.

Les engrais chimiques se présentent souvent sous forme solide et peu soluble dans le sol, nécessitent d'être apportés massivement au moment précis où ces produits de fertilisation sont nécessaires pour la croissance optimale des végétaux. L'excès de ces produits de fertilisation solides apportés au sol et non prélevés par les végétaux reste momentanément dans le sol et est dissout progressivement par les eaux de pluie ou les eaux d'arrosage, entrainant une pollution des cours d'eau et des nappes phréatiques.

Des solutions pour améliorer l'efficacité de la fertilisation des sols sans polluer l'environnement et en particulier les nappes phréatiques tout en favorisant la croissance des végétaux sont donc recherchées.

L'invention vise donc à apporter une solution à ce problème.

Par ailleurs, de nombreux agents phyto-pathogènes sont susceptibles de se développer dans les cultures végétales et à leurs dépens, en diminuant les rendements et la qualité de la production végétale. Parmi ces agents phyto-pathogènes, on trouve de très nombreuses bactéries telles que, par exemple, *Streptomyces scabies* qui est susceptible de causer des dommages aux cultures de pomme de terre. On trouve aussi de nombreux champignons phyto-pathogènes tels que, par exemple, *Fusarium culmorum*, *Botrytis cinerea* responsable notamment de la pourriture grise ou *Phythium ultimum* ou encore *Phaeomoniella chlamydospora*, *Phaeomoniella aleophilum*, *Eutypa lata* ou *Fomitiporia mediterranea* qui sont responsables de pathologies de la vigne.

L'invention vise aussi à apporter une solution pour la protection des végétaux vis-à-vis de certains organismes -bactéries et/ou champignons- qui sont phyto-pathogènes.

Pour ce faire, l'invention concerne une bactérie isolée selon la revendication 1.

La séquence SEQ ID_NO1 est décrite ci-après :

Dans la séquence SEQ ID_NO1 ci-dessus, le symbole « n » en positions 1036 et 1049 de la séquence SEQ ID_NO1 désigne, selon l'IUPAC (« *International Union of Pure and Applied Chemistry* »), l'un quelconque des quatre nucléotides a, t, c ou g. Ainsi, le nucléotide n en position 1036 est choisi dans le groupe formé du nucléotide « a », du nucléotide « t », du nucléotide « g » et du nucléotide « c », et le nucléotide n en position 1049 est choisi, indépendamment du nucléotide en position 1036, dans le groupe formé du nucléotide « a », du nucléotide « t », du nucléotide « g » et du nucléotide « c ».

L'invention concerne donc une bactérie isolée comprenant la séquence d'ADNr 16S identifiée SEQ ID_NO1, ladite bactérie isolée étant choisie dans le groupe formé de :
- la bactérie déposée et enregistrée à la CNCM sous le n° I-4467, et
- des mutants de ladite bactérie déposée et enregistrée à la CNCM sous le n° I-4467.

La bactérie selon l'invention est du genre *Streptomyces.*

Une souche de la bactérie selon une première variante de l'invention a été déposée par le demandeur et enregistrée en date du 7 avril 2011 sous le n° I-4467 auprès de la Collection Nationale de Cultures de Microorganismes (CNCM) de l'Institut Pasteur (dont l'adresse est 25, rue du Docteur Roux, 75724 Paris cedex 15) ayant le statut d'autorité de dépôt internationale selon le traité de Budapest. La bactérie selon l'invention a donc été déposée et enregistrée à la CNCM sous le n° I-4467.

La bactérie selon la première variante de l'invention est une bactérie isolée de tout milieu naturel c'est-à-dire obtenue par isolement à partir de tout milieu naturel d'origine. Elle a été isolée à partir d'un milieu naturel d'origine dans lequel elle préexistait.

Dans une deuxième variante, l'invention concerne une bactérie mutante de la bactérie selon la première variante de l'invention déposée auprès de la CNCM sous le n° I-4467, c'est-à-dire obtenue par mutation de la bactérie selon la première variante de l'invention. Une bactérie mutante selon l'invention comprend aussi une séquence d'ADNr 16S qui est homologue avec la séquence SEQ ID_NO1.

On obtient une telle bactérie mutante par traitement d'une bactérie selon la première variante de l'invention par toute méthode de mutagenèse, notamment choisie dans le groupe formé des méthodes de mutagenèse aléatoire et des méthodes de mutagenèse dirigée.

On réalise un traitement par mutagenèse aléatoire dans lequel on soumet des bactéries selon la première variante de l'invention à au moins un agent mutagène choisi dans le groupe formé des agents mutagènes physiques -notamment en exposant par exemple des bactéries selon l'invention à une lumière ultraviolette ou à une radiation ionisante- et des agents mutagènes chimiques.

Une bactérie mutante présente des différences de séquence d'ADN par rapport à la bactérie selon la première variante de l'invention déposée auprès de la CNCM sous le n° I-4467. Ces différences de séquence d'ADN peuvent affecter des séquences d'ADN distinctes de la séquence de l'ADNᵣ 16S de la bactérie selon l'invention.

Avantageusement et selon l'invention, la bactérie isolée présente une séquence d'ADN codant pour la sous unité béta de l'ARN polymérase (polB) homologue avec la SEQ ID_NO4. La bactérie isolée présente donc la séquence SEQ ID_NO4 à titre de séquence codant pour la sous unité béta de l'ARN polymérase (polB).

Avantageusement et selon l'invention, la bactérie isolée présente une séquence d'ADN codant pour la gyrase (gyrB) homologue avec la SEQ ID_NO5. La bactérie isolée présente donc la séquence SEQ ID_NO5 à titre de séquence codant pour la gyrase (gyrB).Avantageusement et selon l'invention, la bactérie isolée présente une séquence d'ADN codant pour la recombinase (RecA) homologue avec la SEQ ID_NO6. La bactérie isolée présente donc la séquence SEQ ID_NO6 à titre de séquence codant pour la recombinase (RecA).

Avantageusement et selon l'invention, la bactérie isolée présente une séquence d'ADN codant pour la sous unité béta de la tryptophane synthase (trpB) homologue avec la SEQ ID_NO7. La bactérie isolée présente donc la séquence SEQ ID_NO7 à titre de séquence codant pour la tryptophane synthase (trpB).

Avantageusement et selon l'invention, la bactérie isolée présente une séquence d'ADN codant pour la sous unité béta de l'ATP synthase (AtpB) homologue avec la SEQ ID_NO8. La bactérie isolée présente donc la séquence SEQ ID_NO8 à titre de séquence codant pour la sous unité béta de l'ATP synthase (AtpB).

Avantageusement et selon l'invention, la bactérie isolée présente au moins l'une des séquences SEQ ID_NO4, SEQ ID_NO5, SEQ ID_NO6, SEQ ID_NO7 et SEQ ID_NO8. Avantageusement et selon l'invention, la bactérie isolée présente chacune des séquences SEQ ID_NO4, SEQ ID_NO5, SEQ ID_NO6, SEQ ID_NO7 et SEQ ID_NO8.

Avantageusement, une bactérie selon l'invention, est apte à ralentir la croissance d'au moins un micro-organisme, dit micro-organisme cible, choisi dans le groupe formé de *Micrococcus luteus*, *Bacillus subtilis*, *Botrytis cinerae,* de *Fusarium culmorum*, de *Pythium ultimum*, de *Phaeomoniella chlamydospora*, de *Phaeomoniella aelophilum*, de *Eutypa lata*, de *Fomitiporia mediterranea* et de *Botryosphaeria obtusa.*

Une bactérie selon l'invention est caractérisée par tout ou partie des caractéristiques ci-après :
- elle est non pathogène (inoffensive) pour l'homme ;
- c'est une bactérie à Gram positif ;
- c'est une bactérie saprophyte, c'est-à-dire capable de dégrader la matière organique du sol.

L'invention s'étend à toute bactérie produite par un procédé technique à partir d'une bactérie selon la première variante de l'invention et comprenant une séquence d'ADN homologue à 100 % avec la séquence SEQ ID_NO1. Par «procédé technique », on entend tout procédé de culture bactérienne, de sélection bactérienne, d'isolement, de clonage et/ou de modification du matériel génétique de la bactérie -notamment par mutagenèse-.

L'invention s'étend aussi à une souche bactérienne comprenant au moins une bactérie selon l'invention.

Une souche bactérienne selon l'invention est caractérisée par tout ou partie des caractéristiques ci-après :
- elle présente, lorsqu'elle est cultivée sur milieu ISP-2 ou sur milieu Bennett solide, par un mycélium, dit mycélium de substrat, ramifié se développant dans l'épaisseur du milieu solide de couleur variant du jaune-brun au gris-brun selon la composition du milieu solide ;
- elle présente, lorsqu'elle est cultivée sur milieu ISP-2 ou sur milieu Bennett solide, par un mycélium aérien se développant à l'interface air/solide qui est de couleur blanche ;
- elle présente une température optimale de croissance qui est comprise entre 12°C et 37°C, préférentiellement entre 28°C et 30°C ;
- elle présente un pH optimal de croissance compris entre 6 et 8 ;
- elle est apte à utiliser le D-glucose, le mannitol, le lactose, le saccharose, le maltose et la dextrine à titre de source de carbone ;
- elle n'est pas apte à utiliser préférentiellement le galactose, l'inositol, le sorbose, le fructose, l'arabinose, le raffinose, le rhamnose ou la cellulose à titre de source unique de carbone ;
- elle est apte à utiliser les acides aminés, les sels de nitrate et les sels d'ammonium à titre de source d'azote ;
- elle est apte à réduire les nitrates en nitrites, à dégrader l'adénine, le tween 20 et l'acétate de sodium ;
- elle n'est pas apte à hydrolyser l'amidon et à utiliser ses produits d'hydrolyse.

L'invention vise aussi une composition comprenant au moins une bactérie selon l'invention. L'invention vise donc une composition comprenant au moins une bactérie comprenant une séquence d'ADN homologue à 100 % avec la séquence SEQ ID_NO1. Une composition selon l'invention peut comporter, ou non, des bactéries autres que des bactéries selon l'invention. Une composition selon l'invention comprend au moins une bactérie de la souche telle que déposée à la CNCM sous le n° I-4467 et/ou au moins une bactérie mutante de cette souche déposée à la CNCM et comprenant une séquence d'ADN homologue à 100 % avec la séquence SEQ ID_NO1.

La composition selon l'invention peut comprendre des bactéries selon l'invention, lesdites bactéries selon l'invention pouvant être des bactéries vivantes (c'est-à-dire aptes à croître et se multiplier dans un milieu nutritif adapté) ou encore des bactéries mortes (c'est-à-dire des bactéries inactives et inaptes à croître et se multiplier). On obtient de telles bactéries mortes par tout traitement d'inactivation de micro-organismes connu de l'homme du métier, notamment par un traitement thermique (par exemple par chauffage des cellules selon l'invention à la température de 90°C pendant 15 minutes) de dénaturation des protéines des bactéries selon l'invention.

Avantageusement, la composition selon l'invention comprend un nucléotide comprenant la séquence d'ADN homologue à 100 % avec la séquence SEQ ID_NO1.

Avantageusement, la composition selon l'invention peut comprendre à la fois des bactéries vivantes et des bactéries mortes.

Avantageusement et selon l'invention, la composition comprend au moins un milieu propre à la conservation et au développement des bactéries qu'elle contient.

Une composition selon l'invention peut être une composition liquide comprenant des bactéries selon l'invention dans un milieu liquide. Avantageusement, le milieu liquide est un milieu aqueux.

Une composition liquide selon une première variante de l'invention est caractérisée par tout ou partie des caractéristiques ci-après :
- la composition liquide comprend des bactéries en phase de croissance végétative, c'est à dire présentant un métabolisme actif et/ou se multipliant par division cellulaire. Il s'agit donc de bactéries vivantes en phase de croissance végétative ou en phase stationnaire ;
- ledit milieu liquide est un milieu de culture aqueux, notamment choisi dans le groupe formé des milieux complets et des milieux riches comprenant tous les éléments minéraux et des précurseurs organiques nécessaires à la croissance des bactéries selon l'invention, en particulier une source de carbone, une source d'azote, une source de phosphore, des vitamines et des oligoéléments ;
- ledit milieu liquide comprend du D-glucose, un extrait de levure, du phosphate de potassium dibasique, du sulfate d'ammonium, du chlorure de potassium et du glycérol.

Une composition liquide selon une deuxième variante de l'invention, qui peut être combinée à la variante précédente, est caractérisée par tout ou partie des caractéristiques ci-après :
- la composition liquide comprend des bactéries selon l'invention sous forme de spores. On observe la formation de spores lorsque les bactéries selon l'invention -par exemple les bactéries selon la première variante de l'invention déposée auprès de la CNCM sous le n° I-4467 ou, selon la deuxième variante de l'invention, mutantes de ladite bactérie déposée et enregistrée à la CNCM sous le n° I-4467- forment un mycélium primaire se développant à une interface air/solide et initient une croissance aérienne. Des filaments ou « hyphes » aériens non ramifiés se développent alors à partir du mycélium primaire et présentent à leurs extrémités des structures compartimentées précurseurs de spores. Il s'agit donc de bactéries vivantes sous forme de spores ;
- lesdites spores comprenant une séquence d'ADN homologue à 100 % avec la séquence SEQ ID_NO1 ;
- les spores sont lisses et enchaînées en spirales de type « S », les chaînes de spores lisses présentant en moyenne de 10 à 50 spores ;
- ledit milieu liquide comprend du D-glucose, un extrait de levure, une peptone, du carbonate de calcium (CaCO₃) à titre d'inducteur de stress.

On favorise l'obtention de spores de la bactérie selon l'invention -par exemple les bactéries selon la première variante de l'invention déposée auprès de la CNCM sous le n° I-4467 ou, selon la deuxième variante de l'invention, mutantes de ladite bactérie déposée et enregistrée à la CNCM sous le n° I-4467- par culture de bactéries selon l'invention dans un milieu de culture susceptible de générer un stress bactérien, notamment un milieu de culture liquide limité ou carencé en carbone et/ou en azote et/ou en phosphore et/ou en vitamines et/ou en oligoéléments.

Avantageusement, de telles spores sont susceptibles d'être placées dans un milieu nutritif et de réhydratation pour former une population de bactéries selon l'invention en phase végétative.

Une composition liquide selon l'invention peut comprendre des bactéries en phase végétative selon l'invention et des bactéries sous forme de spores selon l'invention. En tout état de cause, les formes végétatives et les spores selon l'invention présentent une séquence d'ADNᵣ 16S homologue à 100 % avec la séquence SEQ ID_NO1.

Une composition selon l'invention peut être une composition à l'état solide comprenant au moins une bactérie selon l'invention.

Dans une première variante, une composition solide selon de l'invention comprend au moins une bactérie selon l'invention et un milieu solide de culture -notamment un milieu solide comprenant une proportion d'agar-agar (E406)-.

Dans une deuxième variante, une composition solide selon de l'invention comprend au moins une bactérie selon l'invention dans un milieu de conservation de bactéries à une température inférieure à -20°C -notamment de l'ordre de -80°C ou à une température proche de la température de l'azote liquide-. L'invention vise donc un milieu de conservation -notamment un milieu de conservation contenant du glycérol et/ou du polyéthylène glycol- comprenant au moins une bactérie selon l'invention.

L'invention vise aussi une composition acellulaire susceptible d'être obtenue par un procédé dans lequel :
- on ensemence et on met en culture au moins une bactérie selon l'invention dans un milieu de culture apte à permettre la croissance de ladite bactérie pendant une durée supérieure à 24 heures et à une température comprise entre 12°C et 37°C, puis ;
- on extrait les bactéries du milieu de culture ou on inactive les bactéries de façon à former la composition acellulaire.

L'invention vise aussi une composition acellulaire comprenant au moins un polynucléotide, ledit polynucléotide comprenant une séquence d'ADN homologue à 100 % avec la séquence SEQ ID_NO1. Une telle composition acellulaire est en particulier un milieu de culture qui a permis la croissance de bactéries selon l'invention et qui est sensiblement exempt desdites bactéries. Une telle composition acellulaire peut ou non présenter de l'ADN comprenant une séquence homologue à 100 % avec la séquence SEQ ID_NO1.

Une telle composition acellulaire selon l'invention obtenue après 24 heures de culture de la souche selon l'invention peut présenter par analyse en HPLC sur colonne en phase inverse une pluralité de pics (P_{24,i}) présentant des temps de rétention (tᵢ) listés dans le tableau 1 ci-après.

**Tableau 1**

| P₂₄,ᵢ | tᵢ (minute) |
|---|---|
| P₂₄,₁ | 3,30 - 3,60 |
| P₂₄,₂ | 3,90 - 4,0 |
| P₂₄,₃ | 5,90 - 6,38 |
| P₂₄,₄ | 10,1 - 10,20 |
| P₂₄,₅ | 13,5 - 13,9 |
| P₂₄,₆ | 21,4 - 21,7 |

Une telle composition acellulaire selon l'invention obtenue après 3 jours (72 heures) de culture de la souche selon l'invention peut présenter en analyse par HPLC sur colonne en phase inverse une pluralité de pics (P_{72,i}) présentant des temps de rétention (tᵢ) et des aires (A_{72,i}) exprimées en valeurs relatives sous lesdits pics (P_{72,i}) listés dans le tableau 2 ci-après.

**Tableau 2**

| P_{72,i} | tᵢ (minute) | A_{72,i} (%) |
|---|---|---|
| P_{72,1} | 3,93 | 3,45 |
| P_{72,2} | 5,30 | 2,02 |
| P_{72,3} | 6,59 | 5,05 |
| P_{72,4} | 8,83 | 1,45 |
| P₇₂,₅ | 9,63 | 3,13 |
| P₇₂,₆ | 11,925 | 9,07 |
| P₇₂,₇ | 12,39 | 1,92 |
| P₇₂,₈ | 12,87 | 1,24 |
| P₇₂,₉ | 13,76 | 1,82 |
| P₇₂,₁₀ | 15,93 | 2,68 |
| P_{72,11} | 16,27 | 2,07 |
| P₇₂,₁₂ | 18,41 | 1,98 |
| P₇₂,₁₃ | 19,38 | 3,15 |
| P₇₂,₁₄ | 20,04 | 6,59 |

On analyse par HPLC l'extrait obtenu par extraction du milieu de culture de la souche selon l'invention par l'acétate d'éthyle, séchage de la solution d'acétate d'éthyle et solubilisation de l'extrait dans le méthanol. La chromatographie HPLC est réalisée sur une colonne Xbridge (Waters, Guyancourt, France) de dimensions 25cm/4,6mm/5µm. L'élution est réalisée par un gradient d'acétonitrile de 20% à 95% dans l'eau avec un débit de 0,8 mL/min. La détection est réalisée à la longueur d'onde de 254 nm.

Avantageusement et selon l'invention, la composition acellulaire est formée d'un milieu de culture bactérien et présente un chromatogramme HPLC, réalisé sur une colonne Xbridge de dimensions 25cm/4,6mm/5µm avec un gradient d'acétonitrile de 20% à 95% dans l'eau avec un débit de 0,8 mL/min, comprenant un premier signal majeur à un temps de rétention de 11,925 min et un deuxième signal majeur à un temps de rétention de 20,04 min.

Dans les conditions d'extraction et d'analyse ci-dessus mentionnées, avantageusement et selon l'invention, la composition acellulaire obtenue après 3 jours (72 heures) de culture de la souche selon l'invention présente un chromatogramme HPLC comprenant un premier signal majeur (P_{72,6}) à un temps de rétention de 11,925 min et un deuxième signal majeur (P_{72,14}) à un temps de rétention de 20,04 min.

Dans les conditions d'analyse ci-dessus mentionnées, avantageusement et selon l'invention, la composition acellulaire obtenue après 3 jours (72 heures) de culture de la souche selon l'invention présente un chromatogramme HPLC comprenant des signaux dits signaux mineurs, à des temps de rétention de 3,93 min, de 5,30 min, de 6,59 min, de 8,83 min, de 9,63 min, de 12,39 min, de 12,87 min, de 13,76 min, de 13,76 min, de 15,93 min, de 16,27 min, de 18,41 min et de 19,38 min.

L'invention vise aussi une composition formée d'un milieu de culture bactérien présentant un chromatogramme HPLC comprenant un premier signal majeur à un temps de rétention de 11,925 min et un deuxième signal majeur à un temps de rétention de 20,04 min. Avantageusement, le chromatogramme HPLC comprend des signaux dits signaux mineurs, à des temps de rétention de 3,93 min, de 5,30 min, de 6,59 min, de 8,83 min, de 9,63 min, de 12,39 min, de 12,87 min, de 13,76 min, de 13,76 min, de 15,93 min, de 16,27 min, de 18,41 min et de 19,38 min.

Les inventeurs ont démontré qu'une telle composition acellulaire qui est obtenue par mise en contact d'au moins une bactérie selon l'invention, mais qui peut être au moins sensiblement exempte de bactéries selon l'invention et/ou qui peut être ou non exempte d'ADN comprenant la séquence homologue à 100% avec la séquence SEQ ID_NO1, présente un effet stimulateur de la croissance de plantes en culture telles que le tournesol, le maïs, le colza, le blé et la tomate et présente aussi une activité antifongique - notamment vis-à-vis de *Botrytis cinerea*- sur les feuilles de vigne.

L'invention s'étend aussi à une composition acellulaire susceptible d'être obtenue par un procédé dans lequel on met en culture au moins une bactérie selon l'invention dans un milieu de culture apte à permettre la croissance de ladite bactérie pendant une durée supérieure à 24 heures.

Avantageusement et selon l'invention, on élimine les bactéries du milieu de culture de façon à former une composition acellulaire au moins sensiblement exempte de bactéries.

Avantageusement et selon l'invention, on élimine l'ADN comprenant la séquence homologue à 100 % avec la séquence SEQ ID_NO1 de la composition acellulaire, par exemple par traitement de la composition acellulaire par une nucléase.

En variante, avantageusement et selon l'invention, on n'élimine pas les bactéries du milieu de culture.

En variante, avantageusement et selon l'invention, on n'élimine pas l'ADN comprenant la séquence homologue à 100 % avec la séquence SEQ ID_NO1 de la composition acellulaire.

Avantageusement, on ensemence et on met en culture les bactéries selon l'invention dans un milieu de culture et pendant une durée suffisante aptes à permettre leur croissance, c'est-à-dire pendant une durée comprise entre 1 jour et 10 jours, à une température comprise entre 12°C et 37°C, préférentiellement comprise entre 28°C et 30°C, en particulier de l'ordre de 30°C, puis on extrait les bactéries du milieu de culture -par exemple par centrifugation du milieu de culture- ou on inactive les bactéries de façon à former la composition acellulaire. Une telle composition acellulaire est donc un milieu de culture qui ne comprend plus les bactéries selon l'invention ou qui comprend des bactéries mortes, qui a été obtenue par mise en contact et culture de bactéries selon l'invention dans un milieu de culture et dans des conditions adaptés à la croissance des bactéries. Une telle composition acellulaire selon l'invention présente des propriétés -notamment des propriétés antifongiques- qui sont proches sinon équivalentes aux propriétés -notamment aux propriétés antifongiques- des bactéries selon l'invention.

L'invention concerne aussi toute utilisation -notamment en agriculture- d'une bactérie ou d'une souche bactérienne ou d'une composition -liquide ou solide- selon l'invention.

L'invention concerne aussi l'utilisation en agriculture d'une composition de traitement d'un végétal comprenant la séquence d'ADN homologue à 100 % avec la séquence SEQ ID_NO1.

En particulier, avantageusement et selon l'invention, on met au contact d'un végétal une composition de traitement comprenant au moins une matière biologique choisie dans le groupe formé :
- d'une bactérie selon l'invention ;
- d'une composition liquide selon l'invention ;
- d'une composition solide selon l'invention ;
- d'une composition acellulaire selon l'invention.

L'invention concerne également une bactérie, une souche bactérienne, une composition (liquide ou solide) bactérienne et une composition acellulaire caractérisées en combinaison par tout ou partie des caractéristiques mentionnées ci-dessus ou ci-après.

D'autres buts, caractéristiques et avantages de l'invention apparaîtront à la lecture de la description suivante, des exemples illustratifs donnés uniquement à titre non limitatif et des figures annexées dans lesquelles :
- la figure 1 est une reproduction de photographies comparatives (la et 1b) de feuilles de vigne montrant l'effet protecteur/curatif vis-à-vis de *Botrytis cinerae* d'un traitement avec une composition de traitement selon l'invention ;
- la figure 2 est une reproduction d'une photographie d'une boite de Pétri montrant l'inhibition par les bactéries selon l'invention de la croissance de *Phaeomoniella chlamydospora* ;
- la figure 3 est une reproduction d'une photographie illustrative de la solubilisation du phosphate de calcium par des bactéries selon l'invention ;
- la figure 4 est une reproduction d'une photographie illustrative de la stimulation de la croissance d'un plant de tournesol par traitement des graines de tournesol avec une composition liquide selon l'invention ;
- la figure 5 est une reproduction d'une photographie illustrative de la stimulation de la croissance d'un plant de maïs par traitement des graines de maïs avec une composition liquide selon l'invention, et ;
- les figures 6a et 6b sont des reproductions de photographies illustratives de la stimulation de la croissance racinaire de plantules de colza par une composition liquide comprenant des bactéries selon l'invention.

La souche bactérienne selon l'invention a été isolée à partir d'un échantillon de la rhizosphère et des racines profondes d'un cep de vigne. Cet échantillon de la rhizosphère a été prélevé à une profondeur comprise entre 10 cm et 50 cm sous la surface du sol et en éliminant la partie superficielle du prélèvement, puis placé dans un sachet stérile fermé hermétiquement et conservé à + 4°C préalablement à l'isolement des souches de *Streptomyces.*

L'échantillon prélevé est mis en suspension dans de l'eau distillée et stérile à raison de 4 g d'échantillon dans 36 mL d'eau sous agitation magnétique à une vitesse de 200 rotations par minute pendant 30 minutes. La suspension obtenue est ensuite placée à la température de 50°C pendant 10 min puis diluée (x10⁷) dans l'eau distillée stérile. 0,1 mL de cette dilution sont étalés sur boîte de pétri contenant un milieu de culture gélosé solide SEA (« Soil Extract Agar ») complémenté avec de l'acide nalidixique (10 mg/L) ou de la novobiocine (25 mg/L) à titre d'antibiotiques et/ou avec de l'antifongique cycloheximide (40 mg/L). Les boîtes de pétri sont ensuite placées en incubateurs à 30°C pendant 21 jours.

On isole les souches actinomycètes par étalement, observation en microscopie optique et reconnaissance de leurs caractéristiques morphologiques. Les souches actinomycètes ainsi isolées et pures sont transférées sur milieu Bennett pour clonage. Les colonies isolées sont maintenues à 4°C pendant deux mois, prélevées et mises en suspension dans du glycérol stérile à 20 % puis placées et conservées à -20°C.

### Séquençage de l'ADNᵣ 16S des bactéries selon l'invention

On analyse la séquence de l'ADNᵣ 16S des bactéries par des méthodes connues de l'homme du métier. Pour ce faire, on cultive les bactéries selon l'invention dans un milieu liquide puis on extrait leur ADN génomique et on amplifie sélectivement la séquence ADNᵣ 16S par PCR (« *Polymerase Chain Reaction* ») en utilisant :
- une amorce universelle « 27f » de séquence SEQ ID_NO2 suivante, « agagtttgat cctggctcag », et ;
- une amorce universelle « 1492r » de séquence SEQ ID_NO3 suivante,
   « ggttaccttg ttacgactt ».

Pour ce faire, on cultive les bactéries selon l'invention sous agitation dans 100 mL de milieu ISP-2 (« ISP Médium 2, International *Streptomyces* Project Yeast Malt Extract Agar ») liquide à 30°C pendant 5 jours. On sépare le mycélium obtenu et le milieu de culture par centrifugation et on lave le mycélium deux fois à l'eau bi-distillée. On réalise une lyse du mycélium dans 500 µl de tampon de lyse (Tris-HCℓ 400 mM, EDTA 60 mM, NaCℓ 150 mM, SDS 1 %, pH 8,0) pendant 10 min à température ambiante. On ajoute ensuite au milieu de lyse 150 µL d'une solution (pH 4,8) obtenue par mélange de 60 mL d'acétate de potassium 5M, 11,5 mL d'acide acétique glacial et 28,5 d'eau distillée et on agite vigoureusement. On centrifuge le milieu de lyse obtenu à 10000 g pendant 1 min. On collecte le surnageant que l'on soumet à une nouvelle étape de centrifugation à 10000 g pendant 1 min. On collecte le surnageant et on lui adjoint un volume égal d'isopropanol. Après agitation, on centrifuge à 10 000g pendant 2 min. L'ADN précipité est lavé avec 300 µL d'alcool éthylique à 70 %, centrifugé puis séché à l'air et dissout dans 50 µL d'eau bi-distillée stérile.

On réalise la PCR avec un nécessaire (InVitrogen) et selon un profil thermique (« Techne Touch Gene PCR Thermal Cycler) » :
- dénaturation à 98°C pendant 3 min ;
- addition de la « Taq-polymérase » ;
- 30 cycles d'amplification comprenant :
   ▪ une phase de chauffage à 94°C pendant 1 min, suivie de ;
   ▪ une phase de chauffage à 52°C pendant 1 min, suivie de ;
   ▪ une phase de chauffage à 72°C pendant 2 min, suivie de ;
- étape d'extension à 72°C pendant 10 min.

Le produit PCR est analysé sur gel d'électrophorèse et révélé par le bromure d'éthidium sous lumière ultraviolette. La comparaison de la séquence SEQ ID_NO1 de l'ADNᵣ 16S des bactéries selon l'invention et des séquences référencées dans les bases de données génomiques au moyen du logiciel « NCBI Blast » ne révèle aucune séquence présentant plus de 99 % d'homologie avec la séquence SEQ ID_NO1 dans les bases de données génomiques.

### EXEMPLE 1 - Production de bactéries selon l'invention.

On ensemence un volume d'un inoculum de bactéries selon l'invention telles que déposées à la CNCM sous le n° I-4467 dans vingt volumes d'un milieu riche. Un tel milieu riche pour la production de biomasse comprend, par exemple, du D-glucose, un extrait de levure, du phosphate de potassium dibasique (K₂HPO₄), du sulfate d'ammonium ((NH₄)₂SO₄), du chlorure de potassium (KCℓ) et du glycérol à pH 7,2. On maintient la culture à la température de 30°C pendant 5 jours.

En variante, il est possible de séparer les bactéries selon l'invention et le milieu de culture de façon à former une composition acellulaire selon l'invention au moins sensiblement exempte de bactéries selon l'invention.

### EXEMPLE 2 - Production de spores selon l'invention.

On ensemence un volume d'un inoculum de bactéries selon l'invention dans vingt volumes de milieu de sporulation. Par exemple, un milieu liquide de sporulation est formé de D-glucose, d'un extrait de levure, d'une peptone, de carbonate de calcium (CaCO₃) et d'eau distillée à pH 7,2.

On maintient la culture à la température de 30°C pendant 6 jours. On obtient une composition selon l'invention comprenant des spores selon l'invention. Il est ensuite possible de séparer les bactéries principalement sous forme de spores selon l'invention et le milieu de culture par tout moyen de séparation liquide/solide, par exemple par centrifugation ou par filtration.

### EXEMPLE 3 - Protection vis-à-vis de pathogènes.

Une composition liquide comprenant des bactéries selon l'invention présente des capacités inhibitrices de la croissance de certaines bactéries telles que, par exemple, *Micrococcus luteus*, *Bacillus subtilis* et *Streptomyces scabies.*

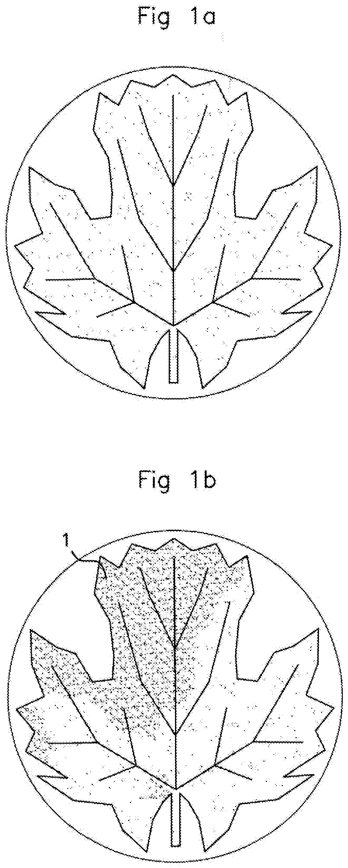

L'application préalable de spores selon l'invention sur des feuilles de vigne permet d'inhiber la germination des spores du champignon *Botrytis cinerae* appliqué ultérieurement sur ces feuilles de vigne et son développement. Une telle application permet aussi de prévenir/éliminer sur les feuilles de vigne prétraitées l'apparition des symptômes (taches brunes sur les feuilles et représentées par un motif grisé (1) sur la figure 1b) dus au champignon *Botrytis cinerae.* Une reproduction de photographies d'une feuille de vigne prétraitée (Fig la) et d'une feuille de vigne non-prétraitée (Fig 1b) avec une composition de spores de la bactérie selon l'invention et infectées par *Botrytis cinerae* est présentée en figure 1. La feuille de la figure la ne présente pas les symptômes d'une infection par *Botrytis cinerae* et est donc protégée vis-à-vis de *Botrytis cinerae* par les spores de la bactérie selon l'invention.

### EXEMPLE 4 - Inhibition de la croissance d'un micro-organisme cible.

On détermine et on quantifie l'activité inhibitrice de la croissance d'un micro-organisme cible par la méthode des cylindres (Bauer et al, 1996) dans laquelle on ensemence les bactéries de la souche déposée à la CNCM sur un milieu Bennett gélosé solide et on place ce milieu ensemencé pendant 5 jours à 30°C de façon à former une composition de traitement solide. On prélève un fragment cylindrique, par exemple un fragment cylindrique de 6 mm de diamètre, de la composition de traitement solide et on dépose ce fragment cylindrique en surface d'un milieu de culture ensemencé avec un micro-organisme cible, par exemple un micro-organisme cible phyto-pathogène. Le milieu de culture du micro-organisme cible peut être par exemple un milieu PDA (« *Potato Dextrose Agar* ») pour les champignons phyto-pathogènes ou un milieu Bennett pour les bactéries phyto-pathogènes. On maintient le fragment cylindrique en surface du milieu de culture ensemencé avec le micro-organisme cible pendant 4 heures à la température de 4°C de façon à permettre la diffusion de composés du milieu de culture de la bactérie selon l'invention dans le milieu ensemencé avec le micro-organisme cible.

On place le milieu ensemencé avec le micro-organisme cible pendant 48 heures à 30°C, puis on mesure le diamètre de la zone d'inhibition de la croissance du micro-organisme cible.

Les bactéries selon l'invention présentent une activité inhibitrice de la croissance de bactéries et/ou de champignons. Le spectre d'activité des bactéries selon l'invention vis-à-vis de la croissance de micro-organismes est présentée au tableau 3 ci-après dans lequel le signe (-) correspond à l'absence d'activité inhibitrice, le signe (+) correspond à un diamètre d'inhibition compris entre 10 mm 15 mm, le signe (++) correspond à un diamètre d'inhibition compris entre 15 mm et 20 mm et le signe (+++) correspond à un diamètre d'inhibition supérieur à 20 mm.

**Tableau 3**

| Micro-organisme cible | Activité inhibitrice |
|---|---|
| *Phaeomoniella chlamydospora* | +++ |
| *Phaeomoniella aelophilum* | +++ |
| *Fomitiporia mediterranea* | ++ |
| *Eutypa lata* | +++ |
| *Botryosphaeria obtusa* | ++ |
| *Botryosphaeria dothidea* | ++ |
| *Botrytis cinerea* | ++ |
| *Verticillium dahliae* | +++ |
| *Fusarium culmorum* | +++ |
| *Pythium ultimum* | ++ |
| *Micrococcus luteus* | +++ |
| *Bacillus subtilis* | +++ |
| *Pseudomonas fluorescens* | - |

Dans ces conditions, le diamètre d'inhibition de la croissance du mycélium de la souche *Botrytis cinerea* par la souche selon l'invention est de 28 mm, le diamètre d'inhibition de la croissance du mycélium de la souche *Fusarium culmorum* est de 30 mm et le diamètre d'inhibition de la croissance du mycélium de la souche *Pythium ultimum* est de 26 mm.

Les bactéries selon l'invention présentent avantageusement des capacités inhibitrices de la croissance d'agents phyto-pathogènes de la vigne comme, par exemple, *Phaeomoniella chlamydospora*, *Phaeomoniella aelophilum*, *Eutypa lata*, *Fomitiporia mediterranea* et *Botryosphaeria obtusa.*

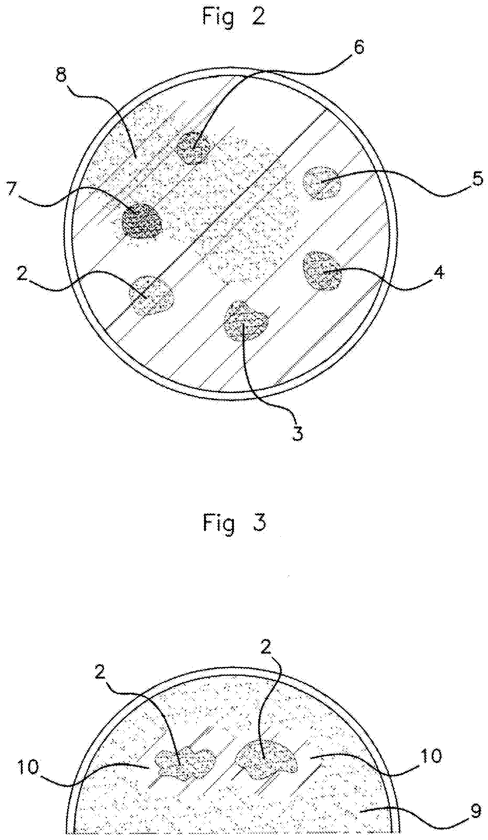

On visualise en figure 2 l'inhibition à grande distance de la croissance du mycélium 8 du champignon *Phaeomoniella chlamydospora* choisi à titre d'agent pathogène du bois de la vigne par les bactéries 2 de la souche selon l'invention par rapport à l'inhibition de la croissance du mycélium 8 de l'agent pathogène par des souches 3, 4 et 5 d'une collection de souches, distinctes de la souche selon l'invention. Il est à noter que les souches 7 et 8 n'inhibent pas la croissance du mycélium 8 de l'agent pathogène. Les bactéries selon l'invention permettent de limiter le développement d'agents phyto-pathogènes bactériens ou fongiques.

### EXEMPLE 5 - Fertilisation

Les bactéries selon l'invention promeuvent la solubilisation d'éléments nutritionnels solides -notamment du phosphore- d'un milieu de culture. Les inventeurs ont observé (figure 3), dans un milieu de culture solide gélosé (9) opacifié par une poudre de phosphate de calcium, la formation d'un halo translucide (10) entourant les colonies (2) de bactéries selon l'invention attestant de la solubilisation du phosphate de calcium par les bactéries. Les bactéries selon l'invention permettent d'augmenter la dissolution -dans la rhizosphère de végétaux- d'un produit de fertilisation solide inassimilable par lesdits végétaux et d'améliorer la nutrition de végétaux.

### EXEMPLE 6 - Stimulation de la croissance du tournesol et du maïs.

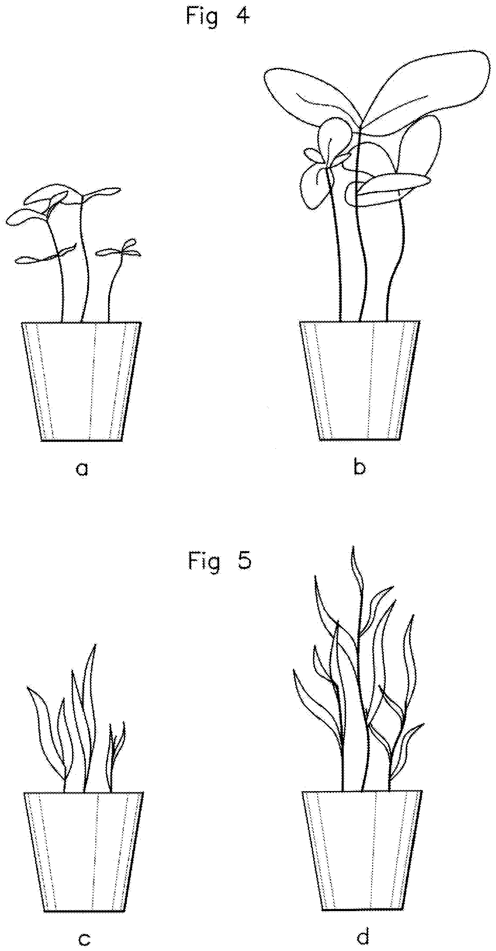

On prépare une composition liquide selon l'invention comprenant des cellules végétatives et des spores de la bactérie selon l'invention que l'on applique par pelliculage sur des graines de tournesol et sur des graines de maïs. La composition liquide comprend entre 1 et 2 g de bactéries par litre de composition, la masse de bactéries étant la masse des bactéries humides. On réalise en parallèle un semis de graines et une culture de plants de tournesol (fig 4a) et de maïs (fig 5c) à titre de contrôles non traités par une composition selon l'invention. Les inventeurs ont aussi observé une stimulation de la croissance initiale des plants de tournesol (figure 4b) et de plants de maïs (figure 5d)-. Les bactéries selon l'invention en application sur les graines permettent de stimuler la croissance de végétaux -en particulier la croissance des parties aériennes de végétaux- tels que le tournesol et le maïs.

### EXEMPLE 7 - Stimulation de la croissance racinaire de plantules de colza.

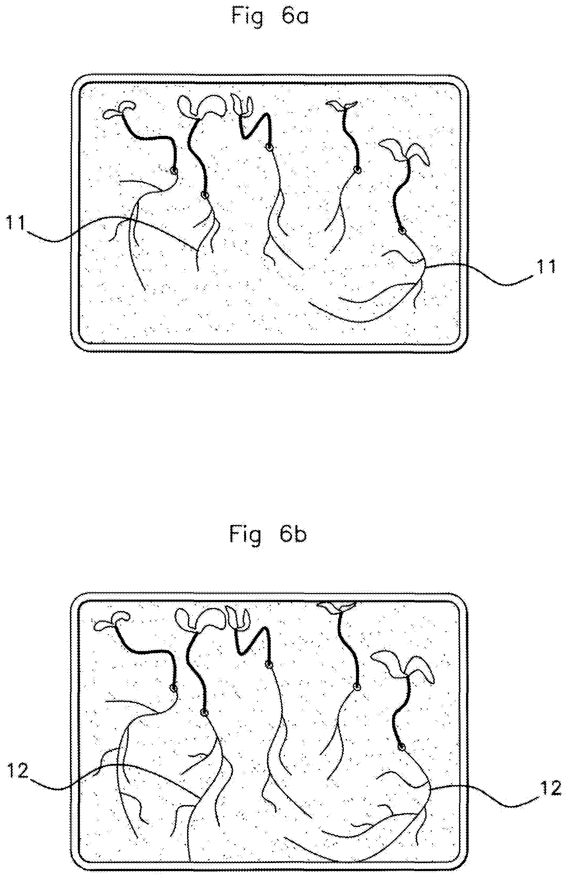

On prépare une composition liquide selon l'invention comprenant des cellules végétatives et des spores de la bactérie selon l'invention par mise en suspension dans l'eau d'un volume d'une pré-culture de la souche déposée à la CNCM dans 60 volumes d'eau. La pré-culture est une culture en phase stationnaire de croissance et comprend de l'ordre de 10 g (en masse humide) de bactéries par litre de pré-culture. On applique la composition liquide selon l'invention sur les graines par mélange et homogénéisation de 16,4 g de graines de colza et de 490 µl de la composition liquide (c'est-à-dire de l'ordre de 1 tonne de graines de colza dans 30 litres de composition liquide) de façon à former un pelliculage de la composition liquide selon l'invention sur les graines de colza. La composition liquide comprend entre 1 et 2 g de bactéries par litre de composition, la masse de bactéries étant la masse des bactéries humides. On réalise le semis des graines de colza enrobées avec la composition selon l'invention sur un milieu de culture « Pikaw ». Après 8 jours de croissance des plantules, on collecte et on pèse les parties racinaires de 12 plantules obtenues à partir de graines traitées avec la composition selon l'invention. La masse des parties racinaires des plantules de colza obtenues à partir de graines traitées avec la composition selon l'invention est de 85 mg et est supérieure à la masse (59 mg) des parties racinaires de 12 plantules de colza obtenues à partir de graines traitées avec une composition exempte de bactéries selon l'invention à titre de contrôle. Les reproductions de photographies de plantules de colza traitées et non traitées sont données respectivement à la figure 6a et à la figure 6b montrent la croissance accrue des parties racinaires 12 de plantules de colza traitées avec la composition selon l'invention en comparaison avec les parties racinaires 11 de plantules de colza traitées avec une composition exempte de bactéries selon l'invention à titre de contrôle. La composition liquide comprenant des bactéries selon l'invention telles que déposées à la CNCM stimule la germination et la croissance de plantules de colza.

### LISTAGE DE SEQUENCES

SEQ ID_NO1
SEQ ID_NO2
   agagtttgat cctggctcag 20
SEQ ID_NO3
   ggttaccttg ttacgactt 19
SEQ ID_NO4
SEQ ID_NO5
SEQ ID_NO6
SEQ ID_NO7
SEQ ID_NO8

## Revendications

1. Bactérie isolée du genre *Streptomyces* choisie dans le groupe formé de :
- la bactérie déposée et enregistrée à la CNCM sous le n° I-4467, et
- des mutants de ladite bactérie déposée et enregistrée à la CNCM sous le n° I-4467 aptes à inhiber la croissance d'au moins un micro-organisme, dit micro-organisme cible, choisi dans le groupe formé de *Botrytis cinerae,* de *Fusarium culmorum*, de *Pythium ultimum*, de *Phaeomoniella chlamydospora*, de *Phaeomoniella aelophilum*, de *Eutypa lata*, de *Fomitiporia mediterranea* et de *Botryosphaeria obtusa*, les mutants de ladite bactérie déposée étant obtenus par mutagenèse de ladite bactérie déposée ;
la bactérie déposée et les mutants de ladite bactérie déposée comprenant une séquence d'ADN, dit ADNᵣ 16S, codant pour l'ARN ribosomal 16S de ladite bactérie, ladite séquence d'ADN étant identique à la séquence SEQ ID_NO1.

2. Bactérie selon la revendication 1, **caractérisée en ce qu'**elle présente une séquence d'ADN codant pour la sous unité béta de l'ARN polymérase (polB) homologue avec la SEQ ID_NO4.

3. Bactérie selon l'une des revendications 1 ou 2, **caractérisée en ce qu'**elle présente une séquence d'ADN codant pour la gyrase (gyrB) homologue avec la SEQ ID_NO5.

4. Bactérie selon l'une des revendications 1 à 3, **caractérisée en ce qu'**elle présente une séquence d'ADN codant pour la recombinase (RecA) homologue avec la SEQ ID_NO6.

5. Bactérie selon l'une des revendications 1 à 4, **caractérisée en ce qu'**elle présente une séquence d'ADN codant pour la sous unité béta de la tryptophane synthase (trpB) homologue avec la SEQ ID_NO7.

6. Bactérie selon l'une des revendications 1 à 5, **caractérisée en ce qu'**elle présente une séquence d'ADN codant pour la sous unité béta de l'ATP synthase (AtpB) homologue avec la SEQ ID_NO8.

7. Composition comprenant au moins une bactérie selon l'une des revendications 1 à 6.

8. Composition selon la revendication 7, **caractérisée en ce qu'**elle est liquide et comprend des bactéries selon l'une des revendications 1 à 6 dans un milieu liquide.

9. Composition selon l'une des revendications 7 ou 8, **caractérisée en ce qu'**elle comprend des bactéries en phase de croissance végétative.

10. Composition selon l'une des revendications 7 à 9, **caractérisée en ce qu'**elle comprend des bactéries sous forme de spores.

11. Composition selon la revendication 7, **caractérisée en ce qu'**elle est à l'état solide.

12. Composition acellulaire obtenue par un procédé dans lequel :
- on ensemence et on met en culture au moins une bactérie selon l'une des revendications 1 à 6 dans un milieu de culture apte à permettre la croissance de ladite bactérie pendant une durée supérieure à 24 heures et à une température comprise entre 12°C et 37°C, puis ;
- on extrait les bactéries du milieu de culture ou on inactive les bactéries de façon à former la composition acellulaire ;
**caractérisée en ce qu'**elle :
- comprend au moins un polynucléotide, ledit polynucléotide comprenant une séquence d'ADN identique à la séquence SEQ ID_NO1 ;
- présente un effet stimulateur de la croissance de plantes en culture telles que le tournesol, le maïs, le colza, le blé et la tomate et une activité antifongique sur les feuilles de vigne.

13. Composition selon la revendication 12 formée d'un milieu de culture bactérien, **caractérisée en ce qu'**elle présente un chromatogramme HPLC, réalisé sur une colonne Xbridge de dimensions 25cm/4,6mm/5µm avec un gradient d'acétonitrile de 20% à 95% dans l'eau avec un débit de 0,8 mL/min, comprenant un premier signal majeur à un temps de rétention de 11,925 min et un deuxième signal majeur à un temps de rétention de 20,04 min.

## Patentansprüche

1. Isolierte Bakterie der Gattung *Streptomyces,* ausgewählt aus der Gruppe, gebildet aus:
- der Bakterie, hinterlegt und registriert in der CNCM unter der Nr. I-4467, und
- Mutanten der Bakterie, hinterlegt und registriert in der CNCM unter der Nr. I-4467, die ausgelegt sind, um das Wachstum von mindestens einem Mikroorganismus, bezeichnet als Ziel-Mikroorganismus, zu hemmen, ausgewählt aus der Gruppe, gebildet aus *Botrytis cinerae, Fusarium culmorum, Pythium ultimum, Phaeomoniella chlamydospora, Phaeomoniella aelophilum, Eutypa* lata, *Fomitiporia mediterranea* und *Botryosphaeria obtusa,* wobei die Mutanten der hinterlegten Bakterie durch Mutagenese der hinterlegten Bakterie erhalten werden;
wobei die hinterlegte Bakterie und die Mutanten der hinterlegten Bakterie eine DNA-Sequenz, bezeichnet als DNAᵣ 16S, umfassen, die für die ribosomale RNA 16S der Bakterie codiert, wobei die DNA-Sequenz identisch mit der Sequenz SEQ ID_NO1 ist.

2. Bakterie nach Anspruch 1, **dadurch gekennzeichnet, dass** sie eine DNA-Sequenz aufweist, die für die Untereinheit beta der RNA-Polymerase (polB) codiert, die mit der SEQ ID_NO4 homolog ist.

3. Bakterie nach einem der Ansprüche 1 oder 2, **dadurch gekennzeichnet, dass** sie eine DNA-Sequenz aufweist, der für die Gyrase (gyrB) codiert, die mit der SEQ ID_NO5 homolog ist.

4. Bakterie nach einem der Ansprüche 1 bis 3, **dadurch gekennzeichnet, dass** sie eine DNA-Sequenz aufweist, die für die Recombinase (RecA) codiert, die mit der SEQ ID_NO6 homolog ist.

5. Bakterie nach einem der Ansprüche 1 bis 4, **dadurch gekennzeichnet, dass** sie eine DNA-Sequenz aufweist, die für die Untereinheit beta der Tryptophansynthase (trpB) codiert, die mit der SEQ ID_NO7 homolog ist.

6. Bakterie nach einem der Ansprüche 1 bis 5, **dadurch gekennzeichnet, dass** sie eine DNA-Sequenz aufweist, die für die Untereinheit beta der ATP-Synthase (AtpB) codiert, die mit der SEQ ID_NO8 homolog ist.

7. Zusammensetzung, umfassend mindestens eine Bakterie nach einem der Ansprüche 1 bis 6.

8. Zusammensetzung nach Anspruch 7, **dadurch gekennzeichnet, dass** sie flüssig ist und Bakterien nach einem der Ansprüche 1 bis 6 in einem flüssigen Medium umfasst.

9. Zusammensetzung nach einem der Ansprüche 7 oder 8, **dadurch gekennzeichnet, dass** sie Bakterien in der vegetativen Wachstumsphase umfasst.

10. Zusammensetzung nach einem der Ansprüche 7 bis 9, **dadurch gekennzeichnet, dass** sie Bakterien in Form von Sporen umfasst.

11. Zusammensetzung nach Anspruch 7, **dadurch gekennzeichnet, dass** sie sich in festem Zustand befindet.

12. Azelluläre Zusammensetzung, erhalten durch ein Verfahren, in dem:
- mindestens eine Bakterie nach einem der Ansprüche 1 bis 6 in ein Kulturmedium geimpft und darin kultiviert wird, das ausgelegt ist, um das Wachstum der Bakterie während einer Dauer von mehr als 24 Stunden und bei einer Temperatur zwischen 12 °C und 37 °C zu ermöglichen, dann;
- die Bakterien aus dem Kulturmedium entfernt oder die Bakterien inaktiviert werden, um die azelluläre Zusammensetzung zu bilden;
**dadurch gekennzeichnet, dass** sie:
- mindestens ein Polynucleotid umfasst, wobei das Polynucleotid eine DNA-Sequenz umfasst, die identisch mit der Sequenz SEQ ID_NO1 ist;
- einen Effekt zur Stimulation des Wachstums von Kulturpflanzen wie z. B. Sonnenblume, Mais, Raps, Weizen und Tomate und eine fungizide Aktivität auf den Blättern des Weins aufweist.

13. Zusammensetzung nach Anspruch 12, gebildet aus einem bakteriellen Kulturmedium, **dadurch gekennzeichnet, dass** sie ein HPLC-Chromatogramm aufweist, durchgeführt auf einer Xbridge-Säule mit den Abmessungen 25 cm/4,6 mm/5 µm mit einem Acetonitrilgradienten von 20 % bis 95 % in Wasser mit einem Durchsatz von 0,8 mL/min, umfassend ein erstes Hauptsignal mit einer Retentionszeit von 11,925 min, und ein zweites Hauptsignal Signal mit einer Retentionszeit von 20,04 min.

## Claims

1. Isolated bacterium of the *Streptomyces* genus chosen from the group composed of:
- the bacterium deposited and registered at the CNCM as No. 1-4467, and
- mutants of said bacterium deposited and registered at CNCM under No. 1-4467 capable of inhibiting growth of at least one micro-organism, called the target micro-organism, chosen from the group composed of *Botrytis cinerae, Fusarium culmorum, Pythium ultimum, Phaeomoniella chlamydospora, Phaeomoniella aelophilum, Eutypa lata*, *Fomitiporia mediterranea* and *Botryosphaeria obtusa,* mutants of said deposited bacterium being obtained by mutagenesis of said deposited bacterium;
the deposited bacterium and the mutants of said deposited bacterium comprising a DNA sequence, called 16S DNAᵣ, coding for 16S ribosomal RNA of said bacterium, said DNA sequence being identical to sequence SEQ ID_NO1.

2. Bacterium according to claim 1, **characterised in that** it has a sequence of DNA coding for the beta sub-unit of RNA polymerase (polB) homologous with SEQ ID_NO4.

3. Bacterium according to either claim 1 or 2, **characterised in that** it has a sequence of DNA coding for the gyrase (gyrB) homologous with SEQ ID_NO5.

4. Bacterium according to one of claims 1 to 3, **characterised in that** it has a sequence of DNA coding for the recombinase (RecA) homologous with SEQ ID_NO6.

5. Bacterium according to one of claims 1 to 4, **characterised in that** it has a sequence of DNA coding for the beta sub-unit of tryptophane synthase (trpB) homologous with SEQ ID_NO7.

6. Bacterium according to one of claims 1 to 5, **characterised in that** it has a sequence of DNA coding for the beta sub-unit of ATP synthase (AtpB) homologous with SEQ ID_NO8.

7. Composition comprising at least one bacterium according to one of claims 1 to 6.

8. Composition according to claim 7, **characterised in that** it is liquid and comprises bacteria according to one of claims 1 to 6 in a liquid medium.

9. Composition according to either claim 7 or 8, **characterised in that** it comprises bacteria in their vegetative growth phase.

10. Composition according to one of claims 7 to 9, **characterised in that** it comprises bacteria in the form of spores.

11. Composition according to claim 7, **characterised in that** it is in the solid state.

12. Acellular composition obtained by a method in which:
- at least one bacterium according to one of claims 1 to 6 is seeded and put into culture in a culture medium capable of supporting growth of said bacterium for a duration of more than 24 hours and at a temperature of between 12°C and 37°C, then;
- bacteria are extracted from the culture medium or the bacteria are deactivated so as to form the acellular composition;
**characterised in that** it:
- comprises at least one polynucleotide, said polynucleotide comprising a DNA sequence identical to sequence SEQ ID_NO1;
- presents an effect stimulating the growth of plants in culture such as sunflower, maize, rapeseed, wheat and tomato and an antifungal activity on vine leaves.

13. Composition according to claim 12, composed of a bacterial culture medium, **characterised in that** it has an HPLC chromatogram made on an Xbridge column with dimensions 25cm/4.6mm/5µm with an acetonitrile gradient of 20% to 95% in water with a flow 0.8 mL/min, comprising a first major signal with a retention time of 11.925 min and a second major signal with a retention time of 20.04 min.
